(19) Europäisches Patentamt / European Patent Office / Office européen des brevets

(11) **EP 2 289 347 B1**

(12) **EUROPEAN PATENT SPECIFICATION**

(45) Date of publication and mention
of the grant of the patent:
**30.03.2016 Bulletin 2016/13**

(21) Application number: **09769822.9**

(22) Date of filing: **17.02.2009**

(51) Int Cl.:
*A23K 10/30* (2016.01)     *A23K 50/00* (2016.01)

(86) International application number:
**PCT/JP2009/000617**

(87) International publication number:
**WO 2009/157112 (30.12.2009 Gazette 2009/53)**

(54) **FEED COMPRISING RAPESEED MEAL**

TIERFUTTERMITTEL ENTHALTEND RAPSSCHROT

ALIMENT POUR ANIMAUX COMPRENANT DE LA FARINE DE GRAINES DE COLZA

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **27.06.2008 JP 2008168843**

(43) Date of publication of application:
**02.03.2011 Bulletin 2011/09**

(60) Divisional application:
**15198565.2**

(73) Proprietor: **J-Oil Mills, Inc.**
**Tokyo 104-0044 (JP)**

(72) Inventors:
• **SAITO, Sanshiro**
**Tokyo 104-0044 (JP)**
• **SATO, Toshiro**
**Tokyo 104-0044 (JP)**
• **KATAOKA, Hisashi**
**Tokyo 104-0044 (JP)**
• **TANI, Kentaro**
**Tokyo 104-0044 (JP)**
• **TSUBOTA, Masafumi**
**Tokyo 104-0044 (JP)**

(74) Representative: **Gallego Jiménez, José Fernando
et al
Ingenias Creaciones, Signos e Invenciones
S.L.P.
Avda. Diagonal 421, 2
08008 Barcelona (ES)**

(56) References cited:
**EP-A1- 1 908 355      JP-A- 2 124 064
JP-A- 4 166 039       JP-A- 5 001 296
JP-A- 10 229 828      JP-A- 63 291 543
JP-A- 2000 316 472    JP-A- 2001 299 236
JP-A- 2002 065 173    JP-A- 2004 321 170
JP-A- 2006 174 790    JP-B1- 3 970 917**

• **V Danielsen ET AL: "Dehulled protein-rich
rapeseed meal as a protein source for early
weaned piglets", Animal Feed Science and
Technology, Vol. 46, 1 January 1994 (1994-01-01),
pages 239-250, XP55008358, DOI:
10.1016/0377-8401(94)90142-2 [retrieved on
2011-09-28]**
• **SOSULSKI F ET AL.: 'Fractionation of rapeseed
meal into flour and hull components' JOURNAL
OF THE AMERICAN OIL CHEMISTS' SOCIETY vol.
58, no. 2, February 1981, pages 96 - 98,
XP008145158**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

1. Field of the Invention

The present invention relates to an inventive feed, particularly, a feed having an improved nutrition value and a method for regulating livestock excreta.

2. Description of the Related Art

At livestock feeding site, it is important that nutrition component of a feed is efficiently taken in by livestock. Further, it is also important to reduce an amount of excreta such as dung because livestock excreta degrades a feed environment and a surrounding living environment, and disposal requires cost.

Conventionally rapeseed meal made of a residue extracted from rapeseed has been utilized as a livestock feed. Although the rapeseed meal is relatively rich in protein and inexpensive, it has had problems of low energy value (nutritive value) and increase in excreta amount when being used as a feed.

As a conventional art to reduce livestock excreta, there have been a fish culture feed to which transglutaminase is added (Japanese Unexamined Patent Application Publication No. 2003-235470, Patent document 1), a livestock/poultry feed containing DATTAN buckwheat (Japanese Unexamined Patent Application Publication No. 2006-174790, Patent document 2), and others such as the treatment of a pig feed with beta-glucanase (JP.2001/299236).

As a method of adjusting components of rapeseed meal, there has been known a method of separating a seed coat after wet-crushing and concentrating protein by a water treatment through an enzyme treatment (Japanese Patent Publication No. 3919866, Patent Document 3) and others such as disclosed in EP 1 908 355, and in Danielsen V et al, Animal Feed Science and Technology 1994; 46: 239-250.

Commonly, although an excreta amount tends to increase when rapeseed meal is used as a feed, a method of regulating the excreta using the rapeseed meal is not studied at all.

Patent document 1: Japanese Unexamined Patent Application Publication No. 2003-235470
Patent document 2: Japanese Unexamined Patent Application Publication No. 2006-174790
Patent Document 3: Japanese Patent Publication No. 3919866

SUMMARY OF THE INVENTION

According to the present invention there is provided a feed according to Claim 1, which is mixed with 1.0 to 30 weight % of rapeseed meal containing 42.5 to 55 weight % as it is of protein and 1 to 6 weight % as it is of crude fiber, wherein the rapeseed meal is obtained by sifting rapeseed meal by a sift with an opening of 500 micrometers (32 mesh) or less. Also according to the present invention there is provided a method according to Claim 2, which is for decreasing livestock excreta and comprises the step of providing livestock with a feed mixed with 1.0 to 30 weight % of the rapeseed meal defined above. It is an object of the present invention to provide a feed capable of improving energy use efficiency of rapeseed meal utilized as a feed and reducing or regulating an amount of excreta occurring in using rapeseed meal.

Inventors of the present invention have developed an innovative feed which is mixed with rapeseed meal having specific characteristics at a specific amount through an earnest research aiming at solving the above problems. In other words, the present invention provides a feed which is mixed with 0.1 to 30% of rapeseed meal containing 41% or more of protein and 8% or less of crude fiber. The inventors also found that a nutrition value of the feed is improved compared with conventional feeds. Therefore, this feed is preferable as a livestock feed having improved nutrition value. Accordingly, the present invention provides an innovative livestock feed having an improved nutrition value, which is mixed with 1.0 to 30% of rapeseed meal containing 42.5 to 55 % of protein and 1 to 6 % or less of crude fiber. Here, % represents weight %. Said rapeseed meal is obtained by sifting rapeseed meal by a sift with an opening of 500 micrometers (32 mesh) or less.

The inventors further found that this feed remarkably regulates and reduces an excreta amount of the livestock. Therefore, this feed is preferable as a feed regulating excreta of the livestock. Accordingly, the present invention also provides a method of decreasing livestock excreta comprising a step of providing livestock with a feed for regulating livestock excreta, which is mixed with 0.1 to 30% of rapeseed meal containing 42.5 to 55 weight % of protein and 1 to 6 weight % or less of crude fiber. Said rapeseed meal is obtained by sifting rapeseed meal by a sift with an opening of 500 micrometers (32 mesh) or less.

Further, the present invention relates to a method of feeding livestock, a method of improving nutrition, and a method of regulating an excreta amount, using the above-described feed.

According to the livestock feed having an improved nutrition value of the present invention, it is possible to obtain an extremely high nutrition value for the livestock. A protein amount ingestible to the livestock increases by increasing protein content as a matter of course. The feed of the present invention increases digestivity of respective components including protein, fat, and carbohydrate, thereby increasing energy value: ME (nitrogen-corrected metabolizable energy) for chicken and TDN (total digestible nutrients) for pig and cow by 10 to 20% or more. A high energy value leads to reduction of the feed. This is advantageous in terms of reduction of feed cost and excreta amount.

With respect to reduction and regulation of excreta by the feed of the present invention, it is not only caused by reduction of indigestible fiber but it effects more than digestivity of administrated rapeseed meal. When the basic feed mixed with ordinary rapeseed meal is administered to ruminant for example, an excreta amount increases. However, according to the feed of the present invention, the amount decreases compared with the basic feed. In a case of administration to poultry and pigs, remarkable excreta regulation effect is found compared with the conventional rapeseed meal. This is because the mixed rapeseed meal is considered to regulate the excreta amount of the total feed. Such the excreta regulation effect beyond expectation further decreases labor and cost of excreta disposal and contributes to improvement of hygienic environment of a stall and an environment surrounding the farm.

BRIEF DESCRIPTION OF THE DRAWINGS

FIG. 1 is a graph of an amount of air-dried excreta per intake amount during a period for administering feed to chickens.
FIG. 2 is a graph showing a digestion rate of respective components, digestible energy, and TDN of a test subject administered to pigs.
FIG. 3 is a graph of an air-dried excreta amount per intake amount during a period for administering feed to pigs.
FIG. 4 is a graph showing a digestion rate of respective components, digestible energy, and TDN of the test subject administered to ruminants.
FIG. 5 is a graph of an air-dried excreta amount per intake amount during a period for administering feed to ruminants.

DESCRIPTION OF PREFERRED EMBODIMENT

A ratio of crude protein and crude fiber in rapeseed meal is important for the rapeseed meal mixed in a feed according to the present invention. Effects are achieved only on condition that both are in specific ranges. In other words, a protein content of a rapeseed meal is 41% or more, preferably a range of 41% and 60%, and according to the present invention in a range of 42.5% and 55%. In a case where the protein content is less than 41%, it is impossible to obtain excreta regulation effect and improvement of energy use efficiency when the rapeseed meal is added to a feed.

Further, a crude fiber content of the rapeseed meal is 8% or less, preferably a range of 1% and 7%, and according to the present invention in a range of 1 % and 6%. In a case where the crude fiber content is beyond 8%, it is impossible to obtain excreta regulation effect and improvement of energy use efficiency when the rapeseed meal is added to a feed.

Further, NDF (neutral detergent fiber) in the fiber is ordinarily 20% or less, preferably 18 % or less. ADF (acid detergent fiber) is ordinarily 15% or less, preferably 13.4% or less. Lignin is ordinarily 4% or less, preferably 3% or less.

As a method of manufacturing the above-described rapeseed meal, a method of sifting treatment is employed. Among others, the sifting treatment by a sift with 32 mesh (open 500 micrometers) or less is used in the present invention because rapeseed meal satisfying both requirements of protein content and crude fiber is easily obtained.

A mixture amount of the rapeseed meal in the feed according to the present invention is 0.1 to 30%, preferably 0.5 to 20%, and more preferably 1 to 18%. In a case where a mixture amount is less than 0.1%, excreta regulation effect is not obtained. On the contrary, in a case where it exceeds 30%, an adverse effect due to excessive addition is expected in some cases.

With respect to ingredients other than the rapeseed meal which is mixed in the feed, ingredients well known to those skilled in the art are usable without particular limitation depending on types of the livestock. Examples of such the ingredients are grains including rice, brown rice, rye, wheat, barley, corn, milo, and soy bean; brans including bran, and defatted rice bran; manufacture residues including corn gluten meal, corn germ meal, corn gluten feed, and corn steep liquor; vegetal oil residues including soybean meal, linseed oil meal, and palm oil meal; oils and fats including soybean oil and fat, powder refined beef fat, and animal oil and fat; inorganic salts including magnesium sulfate, ferric sulfate, copper sulfate, zinc sulfate, potassium iodide, cobalt sulfate, calcium carbonate, tricalcium phosphate, natrium chloride, calcium phosphate, and choline chloride; amino acids including lysine and methionine: vitamins including vitamin A, vitamin B1, vitamin B2, vitamin B6, vitamin B12, vitamin D3, vitamin E, calcium pantothenate, nicotinic-acid amide, and folate; animalized feed including fish flour, powdered nonfat milk, and dry whey; fresh forage; and hay.

Although the feed of the present invention is usable to livestock, fishes, crustacean, and others, application to the livestock is preferable. Examples of livestock are ruminant including cow, goat, and sheep, poultry including chicken, quail, and duck, and pig. Particularly, application to the ruminant is preferable in terms of remarkable reduction of an excreta amount per intake amount.

EXAMPLES

The present invention is described hereinafter in more detail by using examples and comparative examples. However, the present invention is not limited to the examples below.

[Example 1, Comparative Example 1] (Administration Test to Chicken)

(Feed preparation)

Three groups are set up, including a basal diet feeding group where a basal diet shown in Table 1 is provided and two test diet feeding groups where two types of test diets (conventional rapeseed meal-mixed diet and inventive rapeseed meal-mixed diet) are provided. In the test diets, the basal diet and one of two types of test subjects (conventional rapeseed-meal product and inventive rapeseed-meal product) shown in Table 2 are mixed at a ratio of 8 to 2. Here, the basal diet and the test diets are respectively mixed with 0.1% of chrome oxide as an indicator.

[Table 1]

| BASAL DIET COMPOSITION | MIXTURE RATIO (%) |
|---|---|
| CORN | 41.62 |
| MILO | 20.00 |
| DEFATTED RICE BRAN | 15.00 |
| CORN GLUTEN MEAL | 10.00 |
| FISH FLOUR (CP 65%) | 8.00 |
| SOYBEAN OIL | 2.00 |
| CALCIUM CARBONATE | 1.10 |
| DICALCIUM PHOSPHATE | 0.50 |
| SALT | 0.30 |
| VITAMIN GROUP B PREMIX [1] | 0.20 |
| VITAMIN ADE PREMIX [2] | 0.20 |
| MINERAL PREMIX [3] | 0.20 |
| DL-METHIONINE | 0.20 |
| L-LYSINE HYDROCHLORIDE | 0.38 |
| L-TREONINE | 0.05 |
| L-TRIPTOPHAN | 0.05 |
| L-ARGININE | 0.20 |
| SUM | 100.00 |
| 1) g in 1kg: thiamine nitrate 2.0, riboflamin 10.0, pyridoxine hydrochloride 2.0, nicotinic-acid amide 2.0, D-calcium pantothenate 4.35, choline chloride 138.0, folate 1.0<br>2) in 1g: vitamin A oil 10.000IU, vitamin D3 oil 2,000IU, dl-$\alpha$-tocopherol acetate 20mg<br>3) g in 1kg: Mn 80, Zn 50, Fe 6,I1, Cu 0.6 | |

[Table 2]

| | TEST SUBJECT | MOISTURE (%) | CRUDE FAT (%) | CRUDE PROTEIN (%) | CRUDE ASH (%) | CRUDE FIBER (%) |
|---|---|---|---|---|---|---|
| EXAMPLE 1 | INVENTIVE RAPESEED-MEAL PRODUCT* | 9.99 | 2.26 | 45.63 | 7.14 | 5.51 |
| COMPARATIVE EXAMPLE 1 | CONVENTIONAL RAPESEED-MEAL PRODUCT** | 10.30 | 2.50 | 39.31 | 6.49 | 9.06 |
| * Product classified at 48 mesh (open 300 μm) of conventional rapeseed-meal product (Trade name: RAPESEED MEAL manufactured by J-OIL MILLS, INC.) **  ** Conventional rapeseed-meal product, trade name: RAPESEED MEAL manufactured by J-OIL MILLS, INC. | | | | | | |

(Administration method of feed)

Thirty male broiler chicks (chunky) age of about four weeks are prepared. Two test chicks form a group. A group of two test chicks are housed in a cage for metabolism test, and a basal diet is provided to all test chicks for four days so that they become accustomed to a test environment. Subsequently, the basal diet or two types of test diets are assigned to five groups and respective diets are continuously provided for ten days.

A manure mixture which is excreted for five days since six days after starting provision of respective diets is collected twice a day or morning and evening everyday, and every group.

With respect to thus collected manure mixture, a total amount of the mixture is air-dried at approximately 60°C for two days after weighing, an amount for five days is mixed and finely milled, and it is used as a specimen for analysis.

With respect to such the specimen for analysis, nitrogen (N) is analyzed by a Kjeldahl analysis method and gross energy (GE) is measured using a bomb calorimeter.

With respect to the basal diet, two types of the test diets and the collected manure mixture, N and GE are analyzed and chrome oxide is analyzed by a colorimeter method (Bulletin of the National Institute of Animal Industry, No.52, 1992).

(Digestivity and nutritive value)

Nitrogen-corrected metabolizable energy (ME) of a basal diet and two types of test diets are calculated by an index method (Standard Tables of Feed Composition in Japan, 2001) using chrome oxide as an indicator. Next, ME and metabolic rate of two types of test subjects are calculated by a formula described below.

[Formula 1]

$$\text{Test subject ME (Mcal/kg)} = \frac{\text{Test diet ME} - \text{Basal diet ME} \times \text{Basal diet mixture ratio (80\%)}}{\text{Test subject mixture ratio (20\%)}}$$

[Formula 2]

$$\text{Test Subject Metabolic rate (\%)} = \frac{\text{Test subject ME(Mcal/kg)}}{\text{Test subject GE(Mcal/kg)}} \times 100$$

A measurement result of GE, ME and the metabolic rate of the test subject is shown in Table 3.

[Table 3]

| | TEST SUBJECT | GE (Mcal/kg) | ME (Mcal/kg) | Metabolic rate (%) |
|---|---|---|---|---|
| EXAMPLE 1 | INVENTIVE RAPESEED-MEAL PRODUCT | 4.34 | 2.04±0.10 | 47.0±2.3 |

(continued)

| | TEST SUBJECT | GE (Mcal/kg) | ME (Mcal/kg) | Metabolic rate (%) |
|---|---|---|---|---|
| COMPARATIVE EXAMPLE 1 | CONVENTIONAL RAPESEED-MEAL PRODUCT | 4.30 | 1.68±0.12 | 39.1±2.8 |
| Note) Average value ± Standard deviation (n=5) | | | | |

ME and metabolic rate of the conventional rapeseed-meal product mixed in the basal diet substantially corresponds with ME (1.69Mcal/kg) and metabolic rate (40.2%) of the rapeseed meal listed in Standard Tables of Feed Composition in Japan, 2001. On the other hand, with respect to the inventive rapeseed-meal product mixed in the basal diet, GE does not change but ME and metabolic rate increase by about 20%.

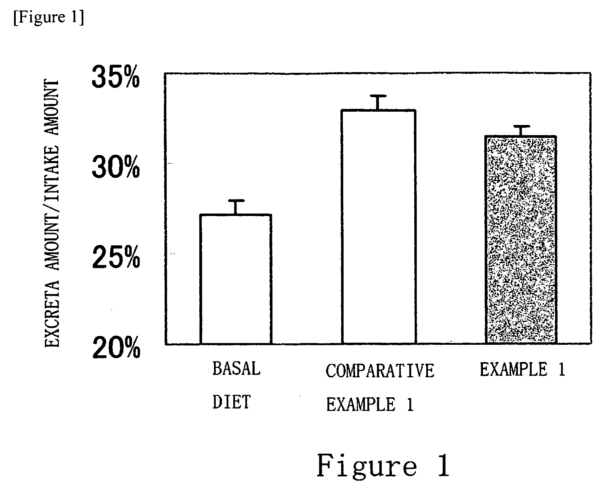

A measurement result of air-dried excreta amount per intake amount for the test period is shown in FIG. 1. The result shows that the excreta amount increases by taking in the diet added with the conventional rapeseed-meal product. On the other hand, in a case where the diet added with the inventive rapeseed-meal product is taken in, the excreta amount is significantly regulated compared with a case of the conventional rapeseed-meal product. Since a difference in amount between the crude fibers of the test subjects is 3.55% (Table 2) and the additive amount to the diet is 20%, a difference in amount between the crude fibers of the diet which is mixed with the conventional rapeseed-meal product and the diet which is mixed with the inventive rapeseed-meal product is only about 0.7% substantially. However, since the excreta rapeseed-meal product, it is shown that the excreta reduction effect of the diet according to the present invention is not simply derived from reduction of crude fibers.

[Examples 2 to 4, Comparative Examples 2 to 3]

A test of administration to chicken which is similar to Example 1 is conducted, except for that rapeseed meal in which protein content and fiber content are different as shown in Table 4 is employed. With respect to ingredient of Examples, rapeseed meal classified at 48 mesh (open 300 $\mu$m) or less is used. Evaluation results on the excreta amount are shown in Table 4 together with evaluations of Example 1 and Comparative Example 1.

[Table 4]

| | RAPESEED MEAL COMPONENT ANALYSIS | | | EXCRETA REDUCTION EFFECT |
|---|---|---|---|---|
| | MOISTURE (%) | CRUDE PROTEIN (%) | CRUDE FIBER (%) | |
| EXAMPLE 1 | 10.0 | 45.6 | 5.5 | Excellent |
| EXAMPLE 2 | 10.8 | 46.2 | 4.7 | Excellent |
| EXAMPLE 3 | 11.8 | 42.9 | 5.5 | Excellent |
| COMPARATIVE EXAMPLE 8 | 12.1 | 41.5 | 6.5 | Good |
| COMPARATIVE EXAMPLE 1 | 10.3 | 39.3 | 9.1 | Bad |
| COMPARATIVE EXAMPLE 2 | 10.1 | 42.0 | 11.1 | Bad |
| COMPARATIVE EXAMPLE 3 | 12.2 | 38.0 | 6.0 | Bad |
| Criteria<br>Excellent: Compared with Comparative Example 1, excreta reduction per intake amount is 1% or more<br>Good: Compared with Comparative Example 1, excreta reduction per intake amount is 0.5% or more<br>Fair: Compared with Comparative Example 1, excreta reduction effect is found<br>Bad: Compared with Comparative Example 1, excreta reduction effect is not found | | | | |

As shown in Table 4, excreta reduction effect is found in 41 % or more of protein content and 8% or less of fiber content. In a case of protein content more than 41.5% and fiber content less than 6.5%, the excreta reduction effect is especially remarkable.

[Examples 5 to 8, Comparative Examples 4 to 5]

A test similar to Example 2 is conducted, except for that the mixture amount of rapeseed meal to the diet changes from 20% to an amount shown in Table 5, and an excreta reduction effect is evaluated. A result is shown in Table 5 together with a result of Example 2.

[Table 5]

|  | Additive amount in diet (%) | Excreta reduction effect |
|---|---|---|
| EXAMPLE 2 | 20.0 | Excellent |
| EXAMPLE 5 | 0.1 | Fair |
| EXAMPLE 6 | 1.0 | Excellent |
| EXAMPLE 7 | 18.0 | Excellent |
| EXAMPLE 8 | 30.0 | Good |
| COMPARATIVE EXAMPLE 4 | 0.05 | Bad |
| COMPARATIVE EXAMPLE 5 | 40.0 | Bad |

Criteria are same as the previous test.

Based on this result, addition effect is not found in a mixture amount of less than 0.1%. The effect is not found also in a mixture amount of 40% or more, and adverse effect due to excessive addition is expected. The effect is especially remarkable in a mixture amount of 1% or more and 20% or less. Further, among others, in a case of mixture amount of 1% or more and 18% or less, the increase amount of excreta is regulated to less than half, compared with excreta increased by addition of rapeseed meal in Comparative Example 1.

[Example 9, Comparison Example 6] (Test of administration to pig)

(Feed preparation)

Three groups are set up, including a basal diet feeding group where a basal diet shown in Table 6 is provided and two test diet feeding groups where two types of test diets (conventional rapeseed meal-mixed diet and inventive rapeseed meal-mixed diet) are provided. In the test diets, a basal diet and one of two types of test subjects (conventional rapeseed-meal product or inventive rapeseed-meal product) shown in Table 7 are mixed at a ratio of 7 to 3. Here, the basal diet and the test diets are respectively mixed with 0.1% of chrome oxide ($Cr_2O_3$) as an indicator.

[Table 6]

| BASAL DIET COMPOSITION | MIXTURE RATIO (%) |
|---|---|
| YELLOW CORN | 51.95 |
| MILO | 20.00 |
| SOYBEAN MEAL | 20.00 |
| DEFATTED RICE BRAN | 5.00 |
| CALCIUM CARBONATE | 1.10 |
| DICALCIUM PHOSPHATE | 0.85 |
| SALT | 0.30 |
| VITAMIN GROUP B PREMIX [1] | 0.20 |
| VITAMIN ADE PREMIX [2] | 0.20 |
| MINERAL PREMIX [3] | 0.20 |
| DL-METHIONINE | 0.05 |

(continued)

| BASAL DIET COMPOSITION | MIXTURE RATIO (%) |
|---|---|
| L-LYSINE | 0.10 |
| L-TRIPTOPHAN | 0.05 |
| SUM | 100.00 |

1) g in 1kg: thiamine nitrate 1.0, riboflamin 7.0, pyridoxine hydrochloride 0.5, nicotinic-acid amide 6.0, D-calcium pantothenate 10.9, choline chloride 57.6
2) in 1g: vitamin A 10,000IU, vitamin D3 2,000IU, dl-$\alpha$-tocopherol acetate 10 mg
3) g in 1kg: Mn 50, Fe 50, Cu 10, Zn 60, I 1

[Table 7]

| | TEST SUBJECT | MOISTURE (%) | CRUDE FAT (%) | CRUDE PROTEIN (%) | CRUDE ASH (%) | CRUDE FIBER (%) |
|---|---|---|---|---|---|---|
| EXAMPLE 9 | INVENTIVE RAPESEED-MEAL PRODUCT | 12.10 | 3.02 | 44.19 | 7.03 | 5.43 |
| COMPARATIVE EXAMPLE 6 | CONVENTIONAL RAPESEED-MEAL PRODUCT | 12.53 | 3.33 | 37.69 | 6.23 | 9.90 |

With respect to the respective diets described above, proximate components (crude protein (CP), crude fat (EE), crude fiber, crude ash, and nitrogen-free extract (NFE)) are analyzed by an analysis method based on Ordinance for Enforcement of the Act on Safety Assurance and Quality Improvement of Feeds (Agriculture and Forestry Ministry Order No.36, July 24,1976). Further, gross energy (GE) is analyzed using a bomb calorimeter.

With respect to the basal diet and two types of test diets, proximate components and GE are measured by the method described above and $Cr_2O_3$ is analyzed by a colorimeter method.

(Feed administration method)

Fifteen LW/D-species castrated piglets aged about 3.5 months to 4 months (weight of 43.8 to 49.5kg, average weight of 46.4kg) are prepared. These pigs are individually housed in a metabolism cage, all test pigs are provided with a basal diet for five days so that they become accustomed to a test environment. Next, the basal diet and two types of test diets are assigned to every five pigs, and respective diets are provided at constant amount for ten days. Feeding amount of the diet is set to about 3% of a weight of respective test pigs when they are divided, and they are provided with an equal amount twice a day, morning and evening.

Fresh excreta excreted are individually collected twice a day, morning and evening, for five days since six days after the start of feeding both diets. Thus collected excreta is weighed each time, a total amount is air-dried for two days at about 60°C, subsequently an amount of five days are mixed and finely milled, and an analysis specimen is thus prepared.

With respect to the excreta, proximate components and GE are measured by the above-described method, and $Cr_2O_3$ is analyzed by a colorimeter method.

(Measurement of digestivity, nutritive value, and excreta amount)

Digestivity of respective components of the basal diet and two types of test samples is calculated by a formula of an index method using $Cr_2O_3$ as an indicator, and subsequently, digestivity, TDN (total digestible nutrients), and DE (digestible energy) of two types of test subjects are calculated by formulae described below.

[Formula 3]

$$\text{digestivity of test subject (\%)} = \frac{\text{digestible component content of test diet} - \text{digestible component content of basal diet} \times \text{mixture ratio of basal diet (70\%)}}{\text{component content of test subject} \times \text{mixture ratio of test subject (30\%)}}$$

[Formula 4]

Test subject TDN (%) = (test subject CP × test subject CP digestivity + test subject crude fat × test subject crude fat digestivity × 2.25 + test subject crude fiber × test subject crude fiber digestivity + test subject NFE × test subject NFE digestivity) / 100

[Formula 5]

Test subject DE (Mcal/kg) = test subject GE × test subject GE digestivity

DE of the inventive rapeseed-meal product which is mixed in the diet is 3.49 ± 0.07Mcal/kg. This corresponds with about 1.14 times DE of the conventional rapeseed-meal product mixed in the diet, 3.05 ± 0.08Mcal/kg.

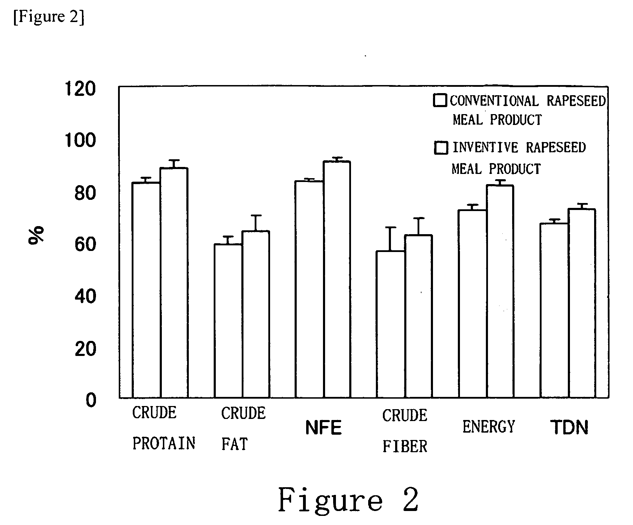

Digestivity, digestible energy, and TDN of respective components in the two types of test subjects are shown in FIG. 2. With respect to the inventive rapeseed-meal product, digestivity increases in all components, and digestible energy and TDN increase as well.

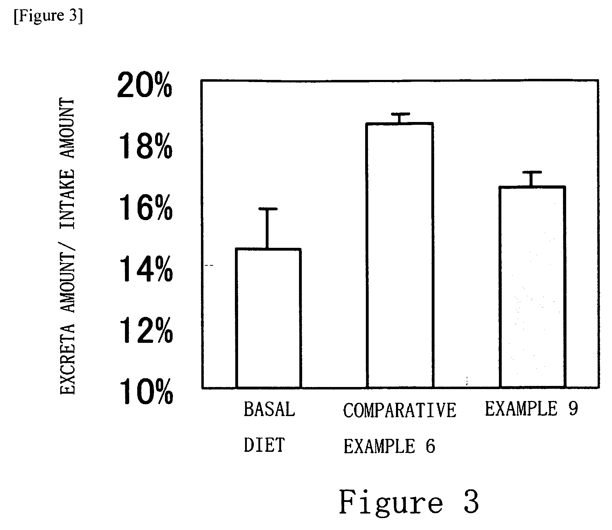

A measurement result of air-dried excreta amount per intake amount during the test period is shown in FIG. 3. Based on this result, the excreta amount increases by taking in the diet added with the conventional rapeseed-meal product. On the other hand, when the diet mixed with the inventive rapeseed-meal product is taken in, the excreta amount is significantly regulated compared with a case of the conventional rapeseed-meal product. Since a difference in amount between the crude fibers of the test subjects is 4.47% and the additive amount to the diet is 30%, a difference in amount between the crude fibers of the diet which is mixed with the conventional rapeseed-meal product and the diet which is mixed with the inventive rapeseed-meal product is only about 1.3% (Table 7) substantially. However, since the excreta amount per intake amount is reduced by about 2.2% from a case of the conventional rapeseed-meal product, it is shown that the excreta reduction effect of the diet according to the present invention is not simply derived from reduction of crude fibers.

[Example 10, Comparative Example 7]

(Test of administration to ruminant)

(Feed preparation)

Three groups are set up, including a basal diet feeding group where a basal diet shown in Table 8 is provided and two test diet feeding groups where two types of test subjects (conventional rapeseed meal- mixed diet and inventive rapeseed meal-mixed diet) are provided. In the test diets, a basal diet and one of two types of test subjects (conventional rapeseed-meal product and inventive rapeseed-meal product) shown in Table 9 are mixed at a ratio of 8 to 2. Here, the basal diet and the test diets are respectively mixed with 0.1% of chrome oxide as an indicator.

[Table 8]

| BASAL DIET COMPOSITION | MIXTURE RATIO (%) |
|---|---|
| CORN | 30.50 |
| MILO | 10.00 |
| DEFATTED RICE BRAN | 7.50 |

(continued)

| BASAL DIET COMPOSITION | MIXTURE RATIO (%) |
|---|---|
| CALCIUM CARBONATE | 0.85 |
| DICALCIUM PHOSPHATE | 0.65 |
| SALT | 0.30 |
| VITAMIN ADE PREMIX [1] | 0.10 |
| MINERAL PREMIX [2] | 0.10 |
| Timothy hay | 50.00 |
| SUM | 100.00 |

1) in 1g: vitamin A 10,000IU, vitamin D3 2,000IU, dl-$\alpha$-tocopherol acetate 10 mg
2) gin 1kg: Mn 50, Fe 50, Cu 10, Zn 60, I 1

[Table 9]

| | MOISTURE (%) | CRUDE PROTEIN (%) | CRUDE FAT (%) | CRUDE ASH (%) | CRUDE FIBER (%) | NDF (%) | ADF (%) | Lignin (%) |
|---|---|---|---|---|---|---|---|---|
| EXAMPLE 10 | 12.5 | 44.0 | 3.6 | 6.8 | 4.9 | 18.0 | 13.4 | 2.7 |
| COMPARATIVE EXAMPLE 7 | 13.8 | 36.9 | 3.0 | 6.3 | 9.9 | 25.8 | 20.8 | 7.6 |

(Feed administration method)

Fifteen castrated goats aged about 16 months to 83 months (weight of 16.7 to 62.2kg, average weight of 32.4kg) are prepared. Test goats are individually housed in a metabolism cage, all the test goats are provided with a basal diet for seven days so that they become accustomed to a test environment. Next, every five goats are provided with the basal diet or one of two types of test diets at constant amount for fifteen days. Feeding amount of the diet is set about 1.5 to 2.5% of a weight of respective test goats when they are divided, and they are provided with an equal amount twice a day, morning and evening.

Excreted feces are individually collected twice a day, morning and evening, for seven days since eight days after the start of providing both diets. Thus collected feces are weighed, an amount of one day is incorporated and air-dried for two days at about 60°C, subsequently an amount of seven days are mixed and finely milled, as an analysis specimen.

With respect to two types of test subjects, concentrated feed, hay, and excreta, proximate components (crude protein (CP), crude fat (EE), crude fiber, crude ash, and nitrogen-free extract (NFE)) are analyzed by an analysis method based on Ordinance for Enforcement of the Act on Safety Assurance and Quality Improvement of Feeds (Agriculture and Forestry Ministry Order No.36, July 24, 1976).

Digestivity of the respective components of the basal diet and the test diets is calculated using a calculation formula of a total excreta collection method, and subsequently digestivity and total digestible nutrients (TDN) of two types of test subjects are calculated using a formula described below. Further, an amount of excreta per diet intake amount is calculated.

[Formula 6]

$$\text{digestivity of test subject (\%)} = \frac{\text{digestible component content of test diet} - \text{digestible component content of basal diet} \times \text{mixture ratio of basal diet (80\%)}}{\text{component content of test subject} \times \text{mixture ratio of test subject (20\%)}}$$

[Formula 7]

$$\text{Test subject TDN (\%)} = (\text{test subject CP} \times \text{test subject CP digestivity} + \text{test subject}$$

$$\text{crude fat} \times \text{test subject crude fat digestivity} \times 2.25 + \text{test subject crude fiber} \times \text{test}$$

$$\text{subject crude fiber digestivity} + \text{test subject NFE} \times \text{test subject NFE digestivity}) / 100$$

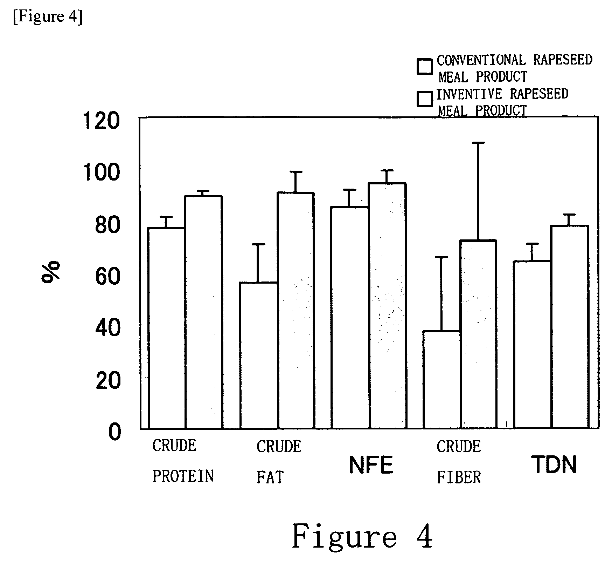

Digestivity and TDN of respective components in the two types of test subjects are shown in FIG. 4. With respect to the inventive rapeseed-meal product, digestivity increases in all components, and TDN also increases to 1.21 times.

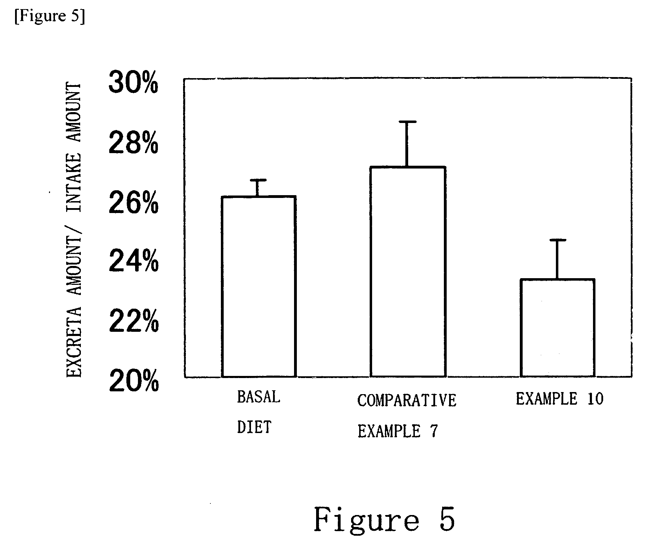

A measurement result of air-dried excreta amount per intake amount during the test period is shown in FIG. 5. Based on this result, the excreta amount increases by taking in the diet added with the conventional rapeseed-meal product. On the other hand, when the diet mixed with the inventive rapeseed-meal product is taken in, the excreta amount is significantly reduced compared with a case of the conventional rapeseed-meal product and the basal diet. Since a difference in amount between the crude fibers of the test subjects is 5% (Table 9) and the additive amount to the diet is 20%, a difference in amount between the crude fibers of diet which is mixed with the conventional rapeseed-meal product and the diet which is mixed with the inventive rapeseed-meal product is only about 1% substantially. However, since the excreta amount per intake amount is reduced by as much as about 3.7 % from a case of the conventional rapeseed-meal product, it is shown that the excreta reduction effect of the diet according to the present invention is not simply derived from reduction of crude fibers.

## Claims

1.  A feed which is mixed with 1.0 to 30 weight % of rapeseed meal containing 42.5 to 55 weight % as it is of protein and 1 to 6 weight % as it is of crude fiber, wherein the rapeseed meal is obtained by sifting rapeseed meal by a sift with an opening of 500 micrometers (32 mesh) or less.

2.  A method of decreasing livestock excreta comprising a step of providing livestock with a feed for regulating livestock excreta which is mixed with 1.0 to 30 weight % of rapeseed meal containing 42.5 to 55 weight % as it is of protein and 1 to 6 weight % as it is of crude fiber, wherein the rapeseed meal is obtained by sifting rapeseed meal by a sift with an opening of 500 micrometers (32 mesh) or less.

## Patentansprüche

1.  Futtermittel, das mit 1,0 bis 30 Gewichtsprozent Rapsschrot gemischt wird, das zu 42,5 bis 55 Gewichtsprozent aus Eiweiß und zu 1 bis 6 Gewichtsprozent aus Rohfasern besteht, wobei das Rapsschrot durch Sieben von Rapsschrot durch ein Sieb mit einer Öffnung von 500 Mikrometern (32 Mesh) oder weniger erhalten wird.

2.  Verfahren zur Verringerung der Ausscheidungen des Viehs, umfassend einen Schritt der Versorgung des Viehs mit einem Futtermittel zur Regulierung der Ausscheidungen des Viehs, das mit 1,0 bis 30 Gewichtsprozent Rapsschrot gemischt wird, das zu 42,5 bis 55 Gewichtsprozent aus Eiweiß und zu 1 bis 6 Gewichtsprozent aus Rohfasern besteht, wobei das Rapsschrot durch Sieben von Rapsschrot durch ein Sieb mit einer Öffnung von 500 Mikrometern (32 Mesh) oder weniger erhalten wird.

## Revendications

1.  Une alimentation qui est mélangée avec 1,0 à 30 % en poids de farine de colza contenant 42,5 à 55 % en poids tel quel de protéine et 1 à 6 % en poids tel quel de cellulose brute, dans laquelle la farine de colza est obtenue en tamisant la farine de colza par un tamiseur avec une ouverture de 500 micromètres (32 mesh) ou moins.

2.  Un procédé de diminution des déjections animales comprenant une étape consistant à procurer au bétail une alimentation pour réguler les déjections animales qui est mélangée avec 1,0 à 30 % en poids de farine de colza contenant 42,5 à 55 % en poids tel quel de protéine et 1 à 6 % en poids tel quel de cellulose brute, dans lequel la farine de colza est obtenue en tamisant la farine de colza par un tamiseur avec une ouverture de 500 micromètres (32 mesh) ou moins.

[Figure 1]

Figure 1

[Figure 2]

Figure 2

[Figure 3]

Figure 3

[Figure 4]

Figure 4

[Figure 5]

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003235470 A
- JP 2006174790 A
- JP 2001299236 A
- JP 3919866 B
- EP 1908355 A

**Non-patent literature cited in the description**

- **DANIELSEN V et al.** *Animal Feed Science and Technology,* 1994, vol. 46, 239-250
- *Bulletin of the National Institute of Animal Industry,* 1992
- *Standard Tables of Feed Composition in Japan,* 2001
- Ordinance for Enforcement of the Act on Safety Assurance and Quality Improvement of Feeds. *Agriculture and Forestry Ministry Order No.36,* 24 July 1976